# EUROPEAN PATENT SPECIFICATION

(11) **EP 1 786 487 B1**
(45) Date of publication and mention of the grant of the patent: **24.11.2010**
(21) Application number: 05793966.2
(22) Date of filing: 02.09.2005
(51) Int. Cl.: A61L 31/04, A61L 31/16, A61L 29/04, A61L 29/16

(54) **MEDICAL DEVICES HAVING SELF-FORMING RATE-CONTROLLING BARRIER FOR DRUG RELEASE**
MEDIZINPRODUKTE MIT SELBSTFORMENDER RATENKONTROLLIERENDER BARRIERE FÜR DIE ARZNEIABGABE
DISPOSITIFS MEDICAUX A BARRIERE DE COMMANDE DE DEBIT AUTO-FORMEE CONÇUS POUR L'ADMINISTRATION DE MEDICAMENTS

(30) Priority: 03.09.2004 US 934844
(43) Date of publication of application: 23.05.2007
(73) Proprietor: Boston Scientific Limited, Christ Church (BB)
(72) Inventor: SCHWARZ, Marlene, C., Aubumdale, MA 02446 (US)
(74) Representative: Jungblut, Bernhard Jakob
(86) International application number: PCT/US2005/031410
(87) International publication number: WO 2006/029012

(56) References cited:
- WO-A-2004/014449
- WO-A-2004/014450
- WO-A-2004/014451
- US-A- 5 702 716

## Description

### FIELD OF THE INVENTION

The present invention relates generally to medical devices which contain polymeric regions for release of therapeutic agents.

### BACKGROUND OF THE INVENTION

Numerous polymer-containing medical devices have been developed for the delivery of therapeutic agents to the body. In accordance with some typical delivery strategies, a therapeutic agent is provided within a polymeric carrier layer and/or beneath a polymeric barrier layer that is associated with a medical device. Once the medical device is placed at the desired location within a patient, the therapeutic agent is released from the medical device at a rate that is dependent upon the nature of the polymeric carrier and/or barrier layer.

Depending on the drug-polymer combination employed, drug delivery coatings for medical devices can release undesirably high levels of the loaded drug over relatively short time intervals.

WO2004/014450 describes an active agent delivery system comprising a hydrophobic cellulose derivative and a polyvinyl homopolymer or copolymer.

WO2004/014449 describes an active agent delivery system including an active agent, a miscible polymer blend that includes a polyurethane and a second polymer having at least one Tg equal or higher than all Tg's of the polyurethane.

WO2004/014451 describes an active agent delivery system comprising an active agent and a miscible polymer blend

### SUMMARY OF THE INVENTION

These and other challenges are addressed by the present invention which, in one aspect, provides a medical device that comprises a rate controlling release region and a therapeutic agent as described in claim 1. The release region comprises a biodisintegrable agent (which can correspond, for example, to a therapeutic agent or to a non-therapeutic agent) and a biostable low Tg polymer. Upon contact of the medical device with a subject (e.g., upon implantation or insertion of the device), the release region becomes depleted, at least at its surface, with respect to the biodisintegrable agent. The low Tg polymer selected naturally migrates, at body temperature, to occupy at least a portion of the volume that is created by the departure of the biodisintegrable agent from the surface. This migration, or consolidation, creates a rate controlling membrane/barrier for therapeutic agent remaining within the device.

An advantage of the present invention is that implantable or insertable medical devices are provided, which display controlled release of a therapeutic agent.

Another advantage of the present invention is that implantable or insertable medical devices are provided, which form a rate controlling barrier layer after implanting and delivering an initial desired amount of therapeutic agent. As a result, such devices can comprise only a single layer, if desired, thereby avoiding the need for providing (e.g., coating or applying) a separate rate controlling barrier layer. In contrast, conventional rate controlling barrier layers are typically coated over an underlying therapeutic-agent containing region, which requires multiple coating steps and may incur additional costs, including materials, labor, machining, and so forth.

These and other aspects, embodiments and advantages of the present invention will become immediately apparent to those of ordinary skill in the art upon review of the Detailed Description and Claims to follow.

### DETAILED DESCRIPTION OF THE INVENTION

A more complete understanding of the present invention is available by reference to the following detailed description of various embodiments of the invention. The detailed description of the embodiments which follows is intended to illustrate but not limit the invention. The scope of the invention is defined by the appended claims.

In one aspect, the invention provides therapeutic-agent releasing medical devices, which contain one or more rate controlling release regions. The one or more release regions in turn contain a biodisintegrable agent and a biostable low Tg polymer. Upon contact (e.g., upon implantation or insertion) of the medical device with a subject (e.g., upon implantation or insertion), the release region becomes depleted at its surface (at least) with respect to the biodisintegrable agent. As the biodisintegrable agent exits the release region, the low Tg polymer migrates (e.g., by flowing, collapsing, coalescence, etc.) to occupy at least a portion of the volume that is created by the departure of the biodisintegrable agent from the surface of the release region. This rearrangement/consolidation of the low Tg polymer creates a rate controlling membrane at the surface of the release region, thereby providing a barrier to release of underlying therapeutic agent.

A "biodisintegrable agent" is one that undergoes dissolution, biodegradation, resorption, erosion, diffusion and/or any other process, whereby the agent is removed from the release region upon being implanted or inserted into a subject. (In contrast, a biostable agent is one that remains associated with the medical device, although it may migrate, over the period in which the device is implanted or inserted into the subject.)

Examples of biodisintegrable agents include both therapeutic and non-therapeutic biodisintegrable agents. Therapeutic biodisintegrable agents can be selected, for example, from biodisintegrable members of the numerous therapeutic agents listed below.

Non-therapeutic biodisintegrable agents include both polymeric and non-polymeric biodisintegrable agents. Examples of non-polymeric biodisintegrable agents include, for example, salts such as potassium chloride, sodium chloride and calcium chloride, sugars such as galactose, glucose and sucrose, cationic lipids, and ionic and nonionic detergents.

Examples of polymeric biodisintegrable agents, which can be of natural or synthetic origin, include the following: cellulosic polymers and copolymers, for example, cellulose ethers such as methylcellulose (MC), hydroxyethylcellulose (HEC), hydroxypropyl cellulose (HPC), hydroxypropyl methyl cellulose (HPMC), methylhydroxyethylcellulose (MHEC), methylhydroxypropylcellulose (MHPC), carboxymethyl cellulose (CMC) and its various salts, including, e.g., the sodium salt, hydroxyethylcarboxymethylcellulose (HECMC) and its various salts, carboxymethylhydroxyethylcellulose (CMHEC) and its various salts, other polysaccharides and polysaccharide derivatives such as starch, dextran, dextran derivatives, chitosan, alginic acid and its various salts, polygalactides, carageenan, varoius gums, including xanthan gum, guar gum, gum arabic, gum karaya, gum ghatti, konjac and gum tragacanth, heparin, glycosaminoglycans and proteoglycans such as hyaluronic acid and its salts, proteins such as gelatin, collagen, albumin, and fibrin, other polymers, for example, polyhydroxyacids such as polylactide, polyglycolide, polyl(lactide-co-glycolide) and poly(ε-caprolactone-co-glycolide), carboxyvinyl polymers and their salts (e.g., carbomer), polyvinylpyrrolidone (PVP), polyacrylic acid and its salts, polyacrylamide, polyacilic acid/acrylamide copolymer, polyhydroxyethylmethacrylate, polyalkylene oxides such as polyethylene oxide, polypropylene oxide and poly(ethylene oxide-propylene oxide) (e.g., Pluronic acid from BASF), polyoxyethylene (polyethylene glycol), polyanhydrides, polyvinylalchol, polyethyleneamine and polypyrridine, additional salts and copolymers beyond those specifically set forth above, and blends of the forgoing (including mixtures of polymers containing the same constitutional units, i.e., monomers, but having substantially different molecular weight distributions). These polymers and copolymers may be of various configurations including, for example, linear, cyclic and branched (e.g., star-shaped, comb-shaped, dendritic, etc.) configurations.

In various embodiments, the biodisintegrable agent of the release region occupies one or more phases that are distinct from the one or more phases occupied by the low-Tg polymer(s). As a specific example, the biodisintegrable agent can correspond to particles that are provided within a matrix formed by the low Tg polymer(s). These particles can contain one or more biodisintegrable agents, for example, one or more therapeutic agents, one or more non-therapeutic agents, or a combination of therapeutic and non-therapeutic agents (e.g., particles containing a therapeutic agent and a biodisintegrable polymer).

As used herein, a "polymer" refers to a grouping of 10 or more constitutional units (i.e., incorporated monomers), commonly 20 or more, 50 or more, 100 or more, 200 or more, 500 or more, or even 1000 or more units. A "low Tg polymer" is a polymer which displays a glass transition temperature (T_{g}), as measured by any of a number of techniques including differential scanning calorimetry (DSC), dynamic mechanical analysis (DMA), or dielectric analysis (DEA), that is below ambient temperature, more typically below 25°C, 0°C, -25°C, or even -50°C. "Ambient temperature" is typically 25°C-45°C, more typically body temperature (e.g., 35°C-40°C). As a result of their low glass transition temperature, low T_{g} polymers are typically elastomeric at ambient temperature.

The low T_{g} polymers for used in conjunction with the present invention may be provided in a variety of configurations, including linear and branched configurations. Branched configurations include star-shaped configurations (e.g., configurations in which three or more chains emanate from a single branch point), comb configurations (e.g., configurations having a main chain and a plurality of side chains) and dendritic configurations (e.g., arborescent and hyperbranched polymers).

The low Tg polymers can contain a single constitutional unit. For example, the low Tg polymers can correspond to low Tg homopolymers. Alternatively, the low Tg polymers can contain multiple constitutional units. For example, the low Tg polymers can correspond to low Tg, random, statistical, gradient or repeating (e.g., alternating) copolymers.

The low Tg polymer is selected such that it will undergo migration upon egress of the biodisintegrable agent from at least the surface of the release region.

Polymers for use as low T_{g} polymers can be selected from appropriate low Tg polymers that are formed from (or having the appearance of being formed from) one or more of the following monomers: acrylic monomers, methacrylic monomers, vinyl ether monomers, cyclic ether monomers, ester monomers, unsaturated hydrocarbon monomers, halogenated unsaturated hydrocarbon monomers, and siloxane monomers. Numerous specific examples from each of these monomer groups are listed below. The T_{g} values given are published values for homopolymers of the listed monomeric unit.

Specific examples of acrylic monomers include: (a) alkyl acrylates such as methyl acrylate (T_{g} 10°C), ethyl acrylate (T_{g} -24°C), propyl acrylate, isopropyl acrylate (T_{g} - 11°C, isotactic), butyl acrylate (T_{g} -54°C), sec-butyl acrylate (T_{g} -26°C), isobutyl acrylate (T_{g} -24°C), cyclohexyl acrylate (T_{g} 19°C), 2-ethylhexyl acrylate (T_{g} -50°C), dodecyl acrylate (T_{g} -3°C) and hexadecyl acrylate (T_{g} 35°C), (b) arylalkyl acrylates such as benzyl acrylate (T_{g} 6°C), (c) alkoxyalkyl acrylates such as 2-ethoxyethyl acrylate (T_{g} -50°C) and 2-methoxyethyl acrylate (T_{g} -50°C), (d) halo-alkyl acrylates such as 2,2,2-trifluoroethyl acrylate (T_{g} -10°C) and (e) cyano-alkyl acrylates such as 2-cyanoethyl acrylate (T_{g} 4°C).

Specific examples of methacrylic monomers include (a) alkyl methacrylates such as butyl methacrylate (T_{g} 20°C), hexyl methacrylate (T_{g} -5°C), 2-ethylhexyl methacrylate (T_{g} -10°C), octyl methacrylate (T_{g} -20°C), dodecyl methacrylate (T_{g} -65°C), hexadecyl methacrylate (T_{g} 15°C) and octadecyl methacrylate (T_{g} -100°C) and (b) aminoalkyl methacrylates such as diethylaminoethyl methacrylate (T_{g} 20°C) and 2-tert-butylaminoethyl methacrylate (T_{g} 33°C).

Specific examples of vinyl ether monomers include (a) alkyl vinyl ethers such as methyl vinyl ether (T_{g} -31°C), ethyl vinyl ether (T_{g} -43°C), propyl vinyl ether (T_{g} -49 °C), butyl vinyl ether (T_{g} -55°C), isobutyl vinyl ether (T_{g} -19°C), 2-ethylhexyl vinyl ether (T_{g} -66°C) and dodecyl vinyl ether (T_{g} -62°C).

Specific examples of cyclic ether monomers include tetrahydrofuran (T_{g} -84°C), trimethylene oxide (T_{g} -78°C), ethylene oxide (T_{g} -66°C), propylene oxide (T_{g} -75°C), methyl glycidyl ether (T_{g} -62°C), butyl glycidyl ether (T_{g} -79°C), allyl glycidyl ether (T_{g}-78°C), epibromohydrin (T_{g} -14°C), epichlorohydrin (T_{g} -22°C), 1,2-epoxybutane (T_{g}-70°C), 1,2-epoxyoctane (T_{g} -67°C) and 1,2-epoxydecane (T_{g} -70°C).

Specific examples of ester monomers (other than acrylates and methacrylates) include ethylene malonate (T_{g} -29°C), vinyl acetate (T_{g} 30°C), and vinyl propionate (T_{g} 10°C).

Specific examples of unsaturated monomers include ethylene, propylene (T_{g} -8 to -13°C), isobutylene (T_{g} -73°C), 1-butene (T_{g} -24°C), trans-butadiene (T_{g} -58°C), 4-methyl pentene (T_{g} 29°C), 1-octene (T_{g} -63°C) and other α-olefins, cis-isoprene (T_{g}-63°C), and trans-isoprene (T_{g} -66°C).

Specific examples of halogenated unsaturated monomers include vinylidene chloride (T_{g} -18°C), vinylidene fluoride (T_{g} -40°C), cis-chlorobutadiene (T_{g} -20°C), and trans-chlorobutadiene (T_{g} -40°C).

Specific examples of siloxane monomers include dimethylsiloxane (T_{g} -127°C), diethylsiloxane, methylethylsiloxane, methylphenylsiloxane (T_{g} -86 °C), and diphenylsiloxane.

As a general rule of thumb, the lower the Tg of the low Tg polymer, the more readily the low Tg block will undergo migration.

Specific examples of low Tg polymers include low Tg alkylene homopolymers and copolymers such as polyisobutylene, low Tg polyurethanes, and low Tg acrylate polymers such as homopolymers and copolymers of butyl acrylate, ethyl acrylate, lauryl acrylate.

As will be appreciated by those or ordinary skill in the art, low Tg polymers may be synthesized according to a number of known methods, including anionic, cationic and radical polymerization methods, such as azobis(isobutyronitrile)- or peroxide-initiated polymerizations and controlled/"living" radical polymerizations such as metal-catalyzed atom transfer radical polymerization (ATRP), stable free-radical polymerization (SFRP), nitroxide-mediated processes (NMP), and degenerative transfer (e.g., reversible addition-fragmentation chain transfer (RAFT)) processes. These methods are well-detailed in the literature and are described, for example, in an article by Pyun and Matyjaszewski, "Synthesis ofNanocomposite Organic/Inorganic Hybrid Materials Using Controlled/"Living" Radical Polymerization," Chem. Mater., 13:3436-3448 (2001).

The release regions of the present invention can correspond, for example, to an entire medical device (e.g., a tubular stent). In other embodiments, the release regions correspond to one or more components of a medical device (e.g., one or more stent struts). In still other embodiments, the release regions correspond to one or more layers disposed over an underlying medical device substrate (e.g., a metallic, ceramic or polymeric substrate). For example, release layers in accordance with the present invention can cover all or a part of an underlying medical device substrate. Multiple release layers can be employed, stacked on top of one another or laterally spaced from one another. As used herein a "layer" of a given material is a region of that material whose thickness is small compared to both its length and width. As used herein a layer need not be planar, for example, taking on the contours of an underlying substrate. Layers can be discontinuous (e.g., patterned). Terms such as "film," "layer" and "coating" may be used interchangeably herein.

In some embodiments, the release region does not contain a therapeutic agent, but rather is disposed over an underlying region that contains the therapeutic agent. In some cases, this underlying region can consist essentially of the therapeutic agent. For example, the underlying region can correspond to a layer of therapeutic agent that is disposed on an underlying substrate. In other cases, the underlying region will contain various agents in addition to the therapeutic agent. For example, the underlying region can contain one or more polymers, which can be the same as or different from the polymer or polymers found in the overlying release region. The therapeutic-agent-containing underlying region can correspond, for example, to a therapeutic-agent-containing polymeric medical device substrate or to a therapeutic-agent-containing polymeric layer disposed over a medical device substrate.

In other embodiments, the release regions themselves contain one or more therapeutic agents. In certain of these embodiments, the therapeutic agent or agents correspond to the biodisintegrable agent of the release region. In certain other embodiments, the release region contains one or more non-therapeutic biodisintegrable agents in addition to one or more therapeutic agents.

Where the release region contains at least one therapeutic agent, it can nonetheless be disposed over an underlying region that comprises at least on additional therapeutic agent as discussed above. The additional therapeutic agent in the underlying region can be can be the same as, or different from, the therapeutic agent in the overlying release region. Moreover, the additional therapeutic agent in the underlying region can have the same, higher, or lower concentration relative to the therapeutic agent in the overlying release region.

Numerous techniques are available for forming the release regions of the present invention. For example, where the selected low Tg polymer has thermoplastic characteristics, and where the biodisintegrable agent is stable a processing temperatures, a variety of standard thermoplastic processing techniques can be used to form release regions, including compression molding, injection molding, blow molding, spinning, vacuum forming and calendaring, as well as extrusion into sheets, fibers, rods, tubes and other cross-sectional profiles of various lengths. Using these and other techniques, entire devices or portions thereof can be made. For example, an entire stent can be extruded using the above techniques. As another example, a coating can be provided by extruding a coating layer onto a pre-existing stent. As yet another example, a coating can be coextruded along with an underlying stent body. If desired, a therapeutic agent can be combined with the low Tg polymer and biodisintegrable agent prior to thermoplastic processing to produce a therapeutic-agent-containing release region, as long as the therapeutic agent is stable at processing temperatures.

As another example, release regions in accordance with the present invention can be formed using solvent-based techniques in which the biodisintegrable agent and low Tg polymer (as well as any other agents, e.g., therapeutic agents, if desired) are first dissolved or dispersed in a solvent system containing one or more solvent species. Subsequently, the resulting mixture is used to form the release region.

Preferred solvent-based techniques include, but are not limited to, solvent casting techniques, spin coating techniques, web coating techniques, solvent spraying techniques, dipping techniques, techniques involving coating via mechanical suspension including air suspension, ink jet techniques, electrostatic techniques, and combinations of these processes. Where the release region is formed using a solvent-based technique, it is typically dried after application to remove the solvents.

The solvent system that is selected for the chosen solvent-based technique contains one or more solvent species. The solvent system is typically a good solvent for the low Tg polymer, although this is not necessarily the case. The solvent system may also be a good solvent for the biodisintegrable agent, but in some embodiments, a solvent system is selected which allows the biodisintegrable agent to remain in particulate form. Where a therapeutic agent is included, the solvent system selected may or may not be a good solvent for the same. The particular solvent species that make up the solvent system may also be selected based on other characteristics including drying rate and surface tension.

In various embodiments, a mixture containing (a) the solvent system, (b) the biodisintegrable agent and low Tg polymer, and (c) further agents, if any, is applied to a substrate to form a release region. For example, the substrate can comprise an implantable or insertable medical device, such as a stent, to which a release region is applied. On the other hand, the substrate can also be, for example, a template, such as a mold, from which the release region is removed after solvent elimination. Such template-based techniques are particularly appropriate for forming simple objects such as sheets, tubes, cylinders and so forth, which can be easily removed from a template substrate.

In other techniques, for example, fiber forming techniques, the release region is formed without the aid of a substrate or template.

Where appropriate, techniques such as those listed above can be repeated or combined to build up a release region to a desired thickness. The thickness of the release region can be varied in other ways as well. For example, in one preferred process, solvent spraying, coating thickness can be increased by modification of coating process parameters, including increasing spray flow rate, slowing the movement between the substrate to be coated and the spray nozzle, providing repeated passes and so forth.

Where a release region is formed over a therapeutic-agent-containing region, the underlying region can also be formed, for example, using thermoplastic and solvent-based techniques such as those discussed above. For example, as noted above, the therapeutic-agent-containing region beneath the release region comprises one or more polymers in some embodiments. As such, the therapeutic-agent-containing region can also be established using thermoplastic and solvent-based techniques (e.g., dipping, spraying, etc.) such as those discussed above. In other embodiments, the therapeutic-agent-containing region beneath the release region is established without an associated polymer matrix. In these instances, for example, the therapeutic agent can simply be dissolved or dispersed in a solvent or liquid, and the resulting solution/dispersion can be contacted with a substrate again using, for example, one or more of the above-described application techniques.

Medical devices for use in conjunction with the present invention include essentially any medical device for which controlled release of a therapeutic agent is desired. Examples of medical devices include implantable or insertable medical devices, for example, catheters (e.g., renal or vascular catheters such as balloon catheters), guide wires, balloons, filters (e.g., vena cava filters), stents (including coronary vascular stents, cerebral, urethral, ureteral, biliary, tracheal, gastrointestinal and esophageal stents), stent grafts, cerebral aneurysm filler coils (including Guglilmi detachable coils and metal coils), vascular grafts, myocardial plugs, patches, pacemakers and pacemaker leads, heart valves, biopsy devices, and any coated substrate (which can comprise, for example, glass, metal, polymer, ceramic and combinations thereof) that is implanted or inserted into the body and from which therapeutic agent is released. Examples of medical devices further include patches for delivery of therapeutic agent to intact skin and broken skin (including wounds); sutures, suture anchors, anastomosis clips and rings, tissue staples and ligating clips at surgical sites; orthopedic fixation devices such as interference screws in the ankle, knee, and hand areas, tacks for ligament attachment and meniscal repair, rods and pins for fracture fixation, screws and plates for craniomaxillofacial repair; dental devices such as void fillers following tooth extraction and guided-tissue-regeneration membrane films following periodontal surgery; and tissue engineering scaffolds for cartilage, bone, skin and other in vivo tissue regeneration.

The medical devices of the present invention include medical devices that are used for either systemic treatment or for the localized treatment of any mammalian tissue or organ. As used herein, "treatment" refers to the prevention of a disease or condition, the reduction or elimination of symptoms associated with a disease or condition, or the substantial or complete elimination of a disease or condition. Preferred subjects are mammalian subjects and more preferably human subjects. Non-limiting examples are tumors; organs including the heart, coronary and peripheral vascular system (referred to overall as "the vasculature"), lungs, trachea, esophagus, brain, liver, kidney, bladder, urethra and ureters, eye, intestines, stomach, pancreas, vagina, uterus, ovary, and prostate; skeletal muscle; smooth muscle; breast; dermal tissue; cartilage; and bone.

Specific examples of medical devices for use in conjunction with the present invention include vascular stents, which deliver therapeutic agent into the vasculature for the treatment of restenosis. In these embodiments, the release region is typically provided over all or a portion of a stent substrate.

As noted above, therapeutic agents may be used singly or in combination in the medical devices of the present invention. "Drugs," "therapeutic agents," "pharmaceutically active agents," "pharmaceutically active materials," and other related terms may be used interchangeably herein. These terms include genetic therapeutic agents, non-genetic therapeutic agents and cells.

Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promoters; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (I) antimicrobial agents such as triclosan, cephalosporins, aminoglycosides and nitrofurantoin; (m) cytotoxic agents, cytostatic agents and cell proliferation affectors; (n) vasodilating agents; (o)agents that interfere with endogenous vasoactive mechanisms; (p) inhibitors of leukocyte recruitment, such as monoclonal antibodies; (q) cytokines; (r) hormones; and (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin.

Preferred non-genetic therapeutic agents include paclitaxel, sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel and Ridogrel.

Exemplary genetic therapeutic agents for use in connection with the present invention include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP) and SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (I) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925 assigned to NeoRx Corporation.

A wide range of therapeutic agent loadings can be used in connection with the medical devices of the present invention, with the therapeutically effective amount being readily determined by those of ordinary skill in the art and ultimately depending, for example, upon the condition to be treated, the age, sex and condition of the patient, the nature of the therapeutic agent, the nature of the release region(s), the nature of the medical device, and so forth.

## Claims

1. A therapeutic-agent-releasing medical device comprising a rate controlling release region that comprises a biodisintegrable agent and a biostable low glass transition temperature polymer that is below ambient temperature, wherein upon contact of said medical device with a subject, the release region becomes depleted with respect to the biodisintegrable agent, and the low glass transition temperature polymer migrates to occupy at least a portion of the volume that is created by the departure of the biodisintegrable agent, thereby forming a barrier layer for therapeutic agent remaining within the device, wherein said biodisintegrable agent is provided in the form of biodisintegrable particles dispersed within said release region.

2. The medical device of claim 1, wherein said release region comprises a polymeric biodisintegrable agent, preferably a water-soluble polymeric biodisintegrable agent, in particular a polymeric biodisintegrable agent selected from soluble polysaccharide-containing polymers, soluble protein-containing polymers, poly(alpha-hydroxy acids), polyacrylamides, polyalkylene oxides, polyanhydrides polyvinylpyrrolidone, alginic acid and its salts, hyaluronic acid and its salts, carboxyvinyl polymers and their salts, and polyacrylic acid and its salts.

3. The medical device of claim 1, wherein said release region comprises a non-polymeric biodisintegrable agent, in particular selected from a sugar or a salt.

4. The medical device of claim 1, wherein said release region comprises a plurality of biodisintegrable agents, or wherein said release region comprises a plurality of low glass transition temperature polymers.

5. The medical device of claim 1, wherein said therapeutic agent is selected from one or more of the group consisting of anti-thrombotic agents, antiproliferative agents, anti-inflammatory agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, and agents that interfere with endogenous vasoactive mechanisms.

6. The medical device of claim 1, wherein said low glass transition temperature polymer is selected from low glass transition temperature polyurethanes, low glass transition temperature silicones, low glass transition temperature acrylates, and low glass transition temperature polyolefins.

7. The medical device of claim 1, wherein said release region is disposed over an underlying region that comprises said therapeutic agent.

8. The medical device of claim 7,
wherein said underlying region is in the form of a layer, or
wherein said underlying region further comprises a polymer.

9. The medical device of claim 1, wherein said release region comprises said therapeutic agent.

10. The medical device of claim 9,
wherein said release region comprises a plurality of therapeutic agents, or
wherein said biodisintegrable agent corresponds to said therapeutic agent, or
wherein said release region comprises a non-therapeutic biodisintegrable agent and said therapeutic agent.

11. The medical device of claim 1, wherein said release region is disposed over an underlying layer that comprises additional therapeutic agent, and wherein said additional therapeutic in said underlying agent is different from or the same as said therapeutic agent in said release region.

12. The medical device of claim 1, wherein said medical device comprises a plurality of release regions.

13. The medical device of claim 1, wherein said release region is in the form of a layer that covers all or a part of an underlying substrate.

14. The medical device of claim 1, wherein said medical device is an implantable or insertable medical device, preferably is selected from a catheter, a guide wire, a balloon, a filter, a stent, a stent graft, a vascular graft, a vascular patch and a shunt, or is adapted for implantation or insertion into the coronary vasculature, peripheral vascular system, esophagus, trachea, colon, biliary tract, urinary tract, prostate or brain.

## Patentansprüche

1. Eine therapeutische-Substanz-freigebende medizinische Vorrichtung umfassend eine Freisetzungsrate kontrollierende Freisetzungsregion, welche eine biozersetzbare Substanz und ein biostabiles Polymer mit niedriger Glasübergangstemperatur, welche unter Umgebungstemperatur ist, umfasst, wobei bei Kontakt der besagten medizinischen Vorrichtung mit einem Subjekt die Freisetzungsregion entleert wird mit Bezug auf die biozersetzbare Substanz und wobei das Polymer mit niedriger Glasübergangstemperatur wandert, um zumindest einen Teil des Volumens, welches durch die Freisetzung der biozersetzbaren Substanz gebildet wird, zu okkupieren, **dadurch** eine Barriereschicht für die therapeutische Substanz bildend, welche in der Vorrichtung verbleibt, wobei besagte biozersetzbare Substanz in der Form von biozersetzbaren Partikeln zur Verfügung gestellt ist, welche innerhalb der Freisetzungsregion dispergiert sind.

2. Die medizinische Vorrichtung von Anspruch 1, wobei die Freisetzungsregion eine polymerische biozersetzbare Substanz umfasst, vorzugsweise eine wasserlösliche polymerische biozersetzbare Substanz, insbesondere eine polymerische biozersetzbare Substanz ausgewählt aus löslichen Polysaccharidenthaltenen Polymeren, löslichen Protein-enthaltenen Polymeren, Poly(alpha-Hydroxysäuren), Polyacrylamiden, Polyalkylenoxiden, Polyanhydriden, Polyvinylpyrrolidonen, Alginsäure und ihre Salze, Hyaluronsäure und ihre Salze, Carboxyvinylpolymere und ihre Salze und Polyacrylsäure und ihre Salze.

3. Die medizinische Vorrichtung von Anspruch 1, wobei die Freisetzungsregion eine nicht-polymerische biozersetzbare Substanz enthält, insbesondere ausgewählt aus einem Zucker oder einem Salz.

4. Die medizinische Vorrichtung gemäß Anspruch 1, wobei besagte Freisetzungsregion eine Mehrzahl von biozersetzbaren Substanzen enthält, oder wobei besagte Freisetzungsregion eine Mehrzahl von Polymeren mit niedriger Glasübergangstemperatur enthält.

5. Die medizinische Vorrichtung gemäß Anspruch 1, wobei besagte therapeutische Substanz ausgewählt ist aus einer oder mehreren der Gruppe bestehend aus antithrombotischen Substanze, anti-proliferativen Substanzen, anti-inflammatorischen Substanzen, antimigratorischen Substanzen, Substanzen, welche die extra-zelluläre Matrixproduktion und Organisation beeinflussen, anti-neoplastischen Substanzen, antimitotischen Substanzen, anästhetischen Substanzen, Antikoagulantien, Gefäßzellwachstumspromotoren, Gefäßzellwachstumsinhibitoren, Cholesterol-senkende Substanzen, gefäßerweiternden Substanzen und Substanzen die mit endogenen vasoaktiven Mechanismen wechselwirken.

6. Die medizinische Vorrichtung gemäß Anspruch 1, wobei besagtes Polymer mit niedriger Glasübergangstemperatur ausgewählt ist aus Polyurethanen mit niedriger Glasübergangstemperatur, Silikonen mit niedriger Glasübergangstemperatur, Acrylaten mit niedriger Glasübergangstemperatur und Polyolefinen mit niedriger Glasübergangstemperatur.

7. Die medizinische Vorrichtung gemäß Anspruch 1, wobei besagte Freisetzungsregion oberhalb einer unterliegenden Region angeordnet ist, welche besagte therapeutische Substanz enthält.

8. Die medizinische Vorrichtung gemäß Anspruch 7, wobei besagte unterliegende Region in der Form einer Schicht ist, oder wobei besagte unterliegende Region zusätzlich ein Polymer umfasst.

9. Die medizinische Vorrichtung gemäß Anspruch 1, wobei besagte Freisetzungsregion besagte therapeutische Substanz enthält.

10. Die medizinische Vorrichtung gemäß Anspruch 9, wobei besagte Freisetzungsregion eine Mehrzahl therapeutischer Substanzen enthält, oder wobei besagte biozersetzbare Substanz mit der besagten therapeutischen Substanz korrespondiert, oder wobei besagte Freisetzungsregion eine nicht-therapeutische biozersetzbare Substanz und besagte therapeutische Substanz enthält.

11. Die medizinische Vorrichtung gemäß Anspruch 1, wobei besagte Freisetzungsregion über einer unterliegenden Region angeordnet ist, welche eine zusätzliche therapeutische Substanz enthält, und wobei besagtes zusätzliches Therapeutikum in besagter unterliegenden Substanz gleich oder verschieden von besagter therapeutischer Substanz in besagter Freisetzungsregion ist.

12. Die medizinische Vorrichtung gemäß Anspruch 1, wobei besagte medizinische Vorrichtung eine Mehrzahl von Freisetzungsregionen umfasst.

13. Die medizinische Vorrichtung gemäß Anspruch 1, wobei besagte Freisetzungsregion in der Form einer Schicht ausgebildet ist, welche ein unterliegendes Substrat vollständig oder ein Teil hiervon bedeckt.

14. Die medizinische Vorrichtung gemäß Anspruch 1, wobei besagte medizinische Vorrichtung eine implantierbare oder einführbare medizinische Vorrichtung ist, vorzugsweise ausgewählt aus einem Katheter, einem Führungsdraht, einem Ballon, einem Filter, einem Stent, einem Stentgraft, einem Gefäßgraft, einem Gefäßpatch und einem Shunt, oder wobei sie adaptiert ist für die Implantierung oder Einführung in Koronargefäße, peripheres Gefäßsystem, Esophagus, Trachea, Kolon, Gallentrakt, Harntrakt, Prostata oder Gehirn.

## Revendications

1. Dispositif médical libérant un agent thérapeutique comprenant une région de libération commandant le débit, qui comprend un agent biodégradable et un polymère biostable à température de transition vitreuse basse, qui est inférieure à la température ambiante, dans lequel après contact du dispositif médical avec un sujet, la région de libération devient appauvrie par rapport à l'agent biodégradable, et le polymère à température de transition vitreuse basse migre pour occuper au moins une partie du volume, qui est créé par le départ de l'agent biodégradable, donc formant une couche de barrière pour l'agent thérapeutique restant dans le dispositif, dans lequel l'agent biodégradable est prévu sous la forme de particules biodégradables dispersées au-dedans de la région de libération.

2. Dispositif médical selon la revendication 1, dans lequel la région de libération comprend un agent biodégradable polymérique, de préférence un agent biodégradable polymérique soluble dans l'eau choisi à partir de polymères solubles comprenant un polysaccharide, polymères solubles comprenant une protéine, poly(alpha-hydroxy acides), polyacrylamides, oxydes de polyalkylène, polyanhydrides, polyvinylpyrrolidone, acide alginique et ses sels, acide hyaluronique et ses sels, polymères de carboxyvinyle et leurs sels, et acide polyacrylique et ses sels.

3. Dispositif médical selon la revendication 1, dans lequel la région de libération comprend un agent biodégradable non polymérique, en particulier choisi à partir d'un sucre ou d'un sel.

4. Dispositif médical selon la revendication 1, dans lequel la région de libération comprend une pluralité d'agents biodégradables, ou dans lequel la région de libération comprend une pluralité de polymères à température de transition vitreuse basse.

5. Dispositif médical selon la revendication 1, dans lequel l'agent thérapeutique est choisi à partir d'un ou plusieurs du groupe consistant en agents anti-thrombotiques, agents anti-prolifératifs, agents anti-inflammatoires, agents anti-migratoires, agents influençant la production et organisation de matrices extracellulaires, agents anti-néoplastiques, agents anti-mitotiques, agents anesthétiques, anti-coagulants, promoteurs de la croissance des cellules vasculaires, inhibiteurs de la croissance des cellules vasculaires, agents abaissant le cholestérol, agents vasodilateurs et agents, qui interfèrent avec des mécanismes vasoactifs endogènes.

6. Dispositif médical selon la revendication 1, dans lequel le polymère à température de transition vitreuse basse est choisi à partir de polyuréthanes à température de transition vitreuse basse, silicones à température de transition vitreuse basse, acrylates à température de transition vitreuse basse et polyoléfines à température de transition vitreuse basse.

7. Dispositif médical selon la revendication 1, dans lequel la région de libération est disposée sur une région positionnée en dessous, qui comprend l'agent thérapeutique.

8. Dispositif médical selon la revendication 7, dans lequel la région positionnée en dessous est sous la forme d'une couche, ou
dans lequel la région positionnée en dessous en outre comprend un polymère.

9. Dispositif médical selon la revendication 1, dans lequel la région positionnée en dessous en outre comprend l'agent thérapeutique.

10. Dispositif médical selon la revendication 9, dans lequel la région de libération comprend une pluralité d'agents thérapeutiques, ou
dans lequel l'agent biodégradable correspond à l'agent thérapeutique, ou
dans lequel la région de libération comprend un agent biodégradable non thérapeutique et l'agent thérapeutique.

11. Dispositif médical selon la revendication 1, dans lequel la région de libération est disposée sur une couche positionnée en dessous, qui comprend un agent thérapeutique additionnel, et dans lequel l'agent thérapeutique additionnel dans l'agent positionné en dessous est différent de ou le même que l'agent thérapeutique dans la région de libération.

12. Dispositif médical selon la revendication 1, dans lequel le dispositif médical comprend une pluralité de régions de libération.

13. Dispositif médical selon la revendication 1, dans lequel la région de libération est sous la forme d'une couche, qui recouvre tout ou une partie d'un substrat positionné en dessous.

14. Dispositif médical selon la revendication 1, dans lequel le dispositif médical est un dispositif médical implantable ou insérable, de préférence est choisi à partir de d'un cathéter, d'un fil de guidage, d'un ballon, d'un filtre, d'un stent, d'un stent à greffer, d'une greffe vasculaire, d'un patch vasculaire et d'un shunt ou est agencé pour l'implantation ou insertion dans le système vasculaire coronaire, le système vasculaire périphérique, l'oesophage, la trachée, le colon, les voies biliaires, les voies urinaires, la prostate ou le cerveau.
